# EUROPÄISCHE PATENTSCHRIFT

(11) **EP 1 974 681 B1**
(45) Veröffentlichungstag und Bekanntmachung des Hinweises auf die Patenterteilung: **16.06.2010**
(21) Anmeldenummer: 08152274.0
(22) Anmeldetag: 04.03.2008
(51) Int. Cl.: A61B 17/72, A61B 17/17

(54) **Implantatset**
Implant set
Ensemble d'implant

(30) Priorität: 28.03.2007 DE 102007016459
(43) Veröffentlichungstag der Anmeldung: 01.10.2008
(73) Patentinhaber: Aesculap AG, 78532 Tuttlingen (DE)
(72) Erfinder: Brunschön, Florian, 78576 Emmingen-Liptingen (DE); Saueressig, Thomas, 78532, Tuttlingen (DE); Stedtfeld, Hans-Werner Privatdozent Dr., 90475 Nürnberg (DE)
(74) Vertreter: Hoeger, Stellrecht & Partner Patentanwälte

(56) Entgegenhaltungen:
- EP-A- 1 495 733
- WO-A-00/13596
- US-A1- 2006 030 859

## Beschreibung

Die vorliegende Erfindung betrifft ein Implantatset, umfassend mindestens zwei unterschiedlich geformte Implantate, welche jeweils einen Schaft, welcher ein proximales und ein distales Ende aufweist, sowie eine am proximalen Ende vorgesehene erste Verbindungseinrichtung zum Verbinden mit einem Zielgerät umfassen, wobei die erste Verbindungseinrichtung eine Verbindungsrichtung definiert.

In der Unfallchirurgie ist es bekannt, Knochenbrüche mithilfe von Nägeln, insbesondere Marknägeln, die teilweise oder vollständig in einen Markraum des verletzten Knochens eingesetzt und mit Befestigungselementen in Form von Schrauben oder Nägeln am Knochen fixiert werden, zu behandeln. Abhängig von der Anatomie eines Patienten werden dabei Implantate unterschiedlicher Länge und/oder unterschiedlicher Form benötigt. Einem Operateur steht üblicherweise ein Implantatset der eingangs beschriebenen Art zur Verfügung, so dass er im Verlauf eines chirurgischen Eingriffs, beispielsweise zur Behandlung einer Oberschenkelhalsfraktur, das jeweils am besten passende Implantat auswählen kann.

Um am Knochen Bohrungen zum Einbringen der Befestigungselemente vorsehen zu können, die fluchtend mit Durchbrechungen am Implantat ausgerichtet sind, wird üblicherweise ein Zielgerät benutzt, welches mit dem Implantat lösbar verbindbar ist. Um das Zielgerät mit unterschiedlich geformten Implantaten verbinden und nutzen zu können, ist es bekannt, entweder spezielle Adapter für die jeweiligen Implantate zur Verbindung mit dem Zielgerät vorzusehen oder mehrere Führungen am Zielgerät, wobei nicht benötigte Führungen durch spezielle Abdeckungen verdeckt werden, um Fehlbohrungen am Knochen zu vermeiden.

In der US 2006/0030859 A1 ist ein Zielgerät für eine Knochenbefestigungsvorrichtung, insbesondere in Form eines Marknagels, offenbart.

Es ist daher Aufgabe der vorliegenden Erfindung, ein Implantatset der eingangs beschriebenen Art so zu verbessern, dass dessen Aufbau und Handhabung vereinfacht und sicherer werden.

Diese Aufgabe wird bei einem Implantatset der eingangs beschriebenen Art erfindungsgemäß dadurch gelöst, dass mindestens zwei Implantate erste Verbindungseinrichtungen aufweisen, welche bezogen auf eine durch einen proximalen Endbereich des jeweiligen Schafts definierte Längsachse unterschiedlich geneigte Verbindungsrichtungen definieren.

Die erfindungsgemäße Weiterbildung eines Implantatsets der eingangs beschriebenen Art ermöglicht es insbesondere, nur ein einziges Zielgerät vorzusehen, welches mit mindestens zwei, vorzugsweise allen Implantaten des Implantatsets verbindbar ist, und zwar derart, dass am Zielgerät nur die tatsächlich erforderlichen Führungen für Bearbeitungswerkzeuge entsprechend einer Zahl von an den Implantaten vorgesehenen Durchbrechungen vorgesehen werden müssen. Dadurch werden Adapter zum Verbinden der Implantate mit dem Zielgerät überflüssig, ebenso spezielle Abdeckungen, mit denen nicht benötigte Führungen am Zielgerät abgedeckt werden können, um Fehlbohrungen zu vermeiden. Insgesamt wird die Handhabung des Implantatsets für einen Operateur besonders einfach, denn er muss sich keine Gedanken machen, wie er welches Implantat des Implantatsets mit dem Zielgerät verbindet. So kann beispielsweise sichergestellt werden, dass entsprechende Führungen für Bearbeitungswerkzeuge am Zielgerät stets mit an den Implantaten vorgesehenen Durchbrechungen fluchten, wenn das Zielgerät mit den Implantaten verbunden ist.

Vorteilhaft ist es, wenn Verbindungsrichtungen unterschiedlicher Implantate relativ zu den durch die jeweiligen proximalen Endbereiche definierten Längsachsen um einen Winkel in einem Bereich von 0° bis 40° geneigt sind. Vorzugsweise liegt der Winkel in einem Bereich von 0° bis 20°. Beispielsweise kann die Verbindungsrichtung eines Implantats relativ zu der vom proximalen Endbereich desselben definierten Längsachse um 0° geneigt sein, das heißt sie können koaxial zueinander ausgerichtet sein. Dies bedeutet insbesondere, dass so das Implantat beispielsweise proximalseitig durch das Zielgerät verlängert werden kann. Je nach Länge und Gestalt des Implantats kann die Neigung der Verbindungsrichtung relativ zur jeweils durch den proximalen Endbereich definierten Längsachse individuell vorgesehen sein und einen beliebigen Wert im Bereich von 0° bis 40° aufweisen.

Günstig ist es, wenn sich Verbindungsrichtungen unterschiedlicher Implantate relativ zu den durch die jeweiligen proximalen Endbereiche definierten Längsachsen um jeweils einen Winkel in einem Bereich von 3° bis 8° unterscheiden. Beispielsweise kann der Neigungswinkel zwischen der Verbindungsrichtung und der durch den proximalen Endbereich definierten Längsachse bei einem Implantat 0° betragen, beim anderen Implantat 5°. Somit unterscheiden sich Verbindungsrichtungen der beiden Implantate relativ zu den durch die jeweiligen proximalen Endbereiche definierten Längsachsen um einen Winkel von 5°. Beispielsweise können sich Verbindungsrichtungen unterschiedlicher Implantate relativ zu den durch die jeweiligen proximalen Endbereiche definierten Längsachsen um jeweils denselben Winkel unterscheiden, beispielsweise einen Winkel von 5°, oder um einen anderen Winkel, welcher im angegebenen Bereich liegt. So ist es auf einfache Weise möglich, kontinuierlich abgestufte Implantate für das Implantatset vorzusehen. Ferner können die Winkelunterschiede so vorgesehen werden, dass häufig vorkommende Anatomien optimal versorgt werden können.

Um Patienten unterschiedlicher Größe optimal behandeln zu können, ist es vorteilhaft, wenn die Schäfte der mindestens zwei unterschiedlich geformten Implantate unterschiedlich lang sind.

Des Weiteren ist es günstig, wenn die Schäfte der mindestens zwei unterschiedlich geformten Implantate unterschiedlich gekrümmt sind. So kann das jeweils zur Patientenanatomie optimal passende Implantat aus dem Implantatset ausgewählt werden. Unter Krümmung ist sowohl eine Krümmung des Implantats auf seiner gesamten Länge zu verstehen als auch eine Krümmung nur im Bereich eines Abschnitts des Implantats, welcher beispielsweise geradlinige Abschnitte desselben verbindet.

Vorzugsweise definieren die Schäfte der mindestens zwei unterschiedlich geformten Implantate unterschiedliche CCD-Winkel (Centrum-Collum-Diaphysen-Winkel). Diese Ausgestaltung ermöglicht es einem Operateur, ein Implantat des Implantatsets auszuwählen, welches optimal an eine Patientenanatomie angepasst ist.

Günstigerweise weisen die mindestens zwei Implantate jeweils mindestens eine Befestigungselementaufnahme auf. Diese kann insbesondere in Form einer Bohrung und/oder einer Gewindebohrung ausgebildet sein. Sie kann auch in Form einer langlochförmigen Durchbrechung vorgesehen sein. Sowohl die Bohrung, die Gewindebohrung als auch die Durchbrechung bilden Befestigungselementaufnahmen, so dass mit einem Befestigungselement das Implantat am Knochen in einer definierten Position gesichert werden kann. Als Befestigungselemente kommen insbesondere Knochenschrauben oder Knochennägel in Frage, die an die jeweilige Befestigungselementaufnahme angepasst sind. Durch die Verwendung von Befestigungselementen können nach der Implantation des Implantats Relativbewegungen des Implantats und des Knochens, an welchem das Implantat festgelegt werden soll, verhindert werden, insbesondere Verdrehungen und Translationsbewegungen des Knochens und des Implantats relativ zueinander.

Besonders einfach wird der Aufbau des Implantatsets, wenn mindestens zwei Befestigungselementaufnahmen vorgesehen sind, welche relativ zueinander bei den mindestens zwei unterschiedlich geformten Implantaten identisch ausgerichtet sind. Dies gestattet es, nur ein Zielgerät vorzusehen, welches beispielsweise zwar unterschiedlich mit den Implantaten in Bezug auf die definierten Verbindungsrichtungen verbunden werden kann, jedoch eine eindeutige Beziehung zwischen einer am Zielgerät vorgesehenen Führung und einer oder mehrerer Befestigungselementaufnahmen, zum Beispiel in Form von Bohrungen und/oder Gewindebohrungen, vorgibt.

Um das Implantat auf einfache Weise dauerhaft sicher und fest mit einem Knochen verbinden zu können, ist es günstig, wenn die mindestens eine Befestigungselementaufnahme, zum Beispiel eine Bohrung und/oder eine Gewindebohrung, an einem proximalen und/oder einem distalen Endbereich des Schafts vorgesehen ist.

Zum Verbinden des Zielgeräts mit den Implantaten des Implantatsets ist es vorteilhaft, wenn die erste Verbindungseinrichtung mindestens ein erstes Verbindungselement umfasst. Es kann insbesondere in optimaler Weise an ein vorzugsweise korrespondierendes Verbindungselement, welches am Zielgerät vorgesehen ist, angepasst sein, um eine optimale Verbindung des Zielgeräts mit den Implantaten in den jeweiligen Verbindungsstellungen zu ermöglichen.

Besonders einfach wird der Aufbau des Implantatsets, wenn das mindestens eine erste Verbindungselement ein mit einem Innengewinde versehenes Sackloch umfasst. Ein solches erstes Verbindungselement lässt sich insbesondere einfach und sicher mit einem proximalen Endbereich eines Implantats des Implantatsets ausbilden.

Gemäß einer bevorzugten Ausführungsform der Erfindung kann vorgesehen sein, dass eine vom ersten Verbindungselement definierte Längsachse relativ zu einer durch den proximalen Endbereich des jeweiligen Schafts definierte Längsachse bei mindestens zwei Implantaten unterschiedlich geneigt ist. So lassen sich auf einfache Weise unterschiedliche Verbindungsrichtungen bei unterschiedlichen Implantaten des Implantatsets ausbilden. Ferner kann so zudem das erste Verbindungselement auch direkt die Verbindungsrichtung für das Implantat definieren. Umgekehrt kann durch entsprechende Neigung der Längsachse des ersten Verbindungselements relativ zu einer durch den proximalen Endbereich des jeweiligen Schafts definierte Längsachse die Verbindungsrichtung in gewünschter Weise definieren.

Grundsätzlich wäre es denkbar, die von den ersten Verbindungselementen der Implantate definierten Längsachsen unter einem beliebigen Winkel relativ zu durch proximale Endbereiche der Implantate definierte Längsachsen vorzusehen. Besonders vorteilhaft ist es jedoch, wenn die von den ersten Verbindungselementen der Implantate definierten Längsachsen relativ zu den durch die jeweiligen proximalen Endbereiche definierten Längsachsen um einen Winkel in einem Bereich von 0° bis 40° geneigt sind. Dies bedeutet beispielsweise, dass die Längsachse des ersten Verbindungselements relativ zur Längsachse des proximalen Endbereichs des Implantats um 0° geneigt sein kann, das heißt die beiden Längsachsen sind koaxial zueinander ausgerichtet oder verlaufen parallel zueinander. Bei anderen Implantaten des Implantatsets können die Längsachsen jedoch auch um einen Winkel geneigt sein, der einen Wert in einem Bereich von 0° bis 40° aufweist, beispielsweise 5°, 10°, 15° oder aber auch 40°. Der jeweilige Winkel kann insbesondere in Korrelation zu einer Länge und/oder einer Krümmung des Implantats stehen. Beispielsweise kann der Winkel um so größer sein, je stärker gekrümmt und/oder je länger das Implantat ist oder auch umgekehrt.

Zur Ausbildung eines Implantatsets, welches an häufig vorkommende Anatomien von Patienten optimal angepasste Implantate umfasst, ist es vorteilhaft, wenn sich die von den ersten Verbindungselementen definierten Längsachsen unterschiedlicher Implantate relativ zu den durch die jeweiligen proximalen Endbereiche definierten Längsachsen um jeweils einen Winkel in einem Bereich von 3° bis 8° unterscheiden. Die von den ersten Verbindungselementen definierten Längsachsen unterschiedlicher Implantate unterscheiden sich beispielsweise um einen Winkel von 5°, wenn zum Beispiel die Längsachse des ersten Verbindungselements eines Implantats relativ zur Längsachse des proximalen Endbereichs dieses Implantats um 10° geneigt ist und dieser Winkel bei einem anderen Implantat des Implantatsets 15° beträgt. Eine Abstufung von Winkeln zwischen den jeweiligen Implantaten kann konstant sein, das heißt es können beispielsweise zwischen allen Implantaten Unterschiede von 5°, 6°, 7° oder 8° vorgesehen sein. Die unterschiedlichen Winkel können jedoch auch abhängig von einer Neigung der Längsachse des ersten Verbindungselements relativ zu einer durch den proximalen Endbereich des Implantats definierten Längsachse vorgegeben werden, insbesondere derart, dass der Winkel um so größer ist, je größer eine Abweichung der Längsachse des ersten Verbindungselements von der durch den proximalen Endbereich des Implantats definierten Längsachse ist.

Um eine optimale Verbindung der Implantate des Implantatsets mit dem Zielgerät sicherzustellen, ist es vorteilhaft, wenn eine in proximaler Richtung weisende Kontur des Schafts korrespondierend zu einem am Zielgerät vorgesehenen Anlagebereich ausgebildet ist. Diese korrespondierende Ausbildung kann insbesondere derart vorgesehen sein, dass eine kraft- und/oder formschlüssige Verbindung oder Anlage zwischen dem Implantat und dem Zielgerät ermöglicht wird.

Auf einfache Weise lässt sich eine definierte Anlage des Implantats an das Zielgerät dadurch erreichen, dass die Kontur zwei relativ zueinander um einen Neigungswinkel geneigte Flächenbereiche aufweist. So kann eine Montage des Zielgeräts am Implantat vereinfacht und eine hierfür benötigte Zeit gegenüber herkömmlichen Implantaten deutlich verringert werden.

Besonders einfach wird der Aufbau des Implantatsets, wenn der Neigungswinkel einen Wert in einem Bereich von 90° bis 150° aufweist. Vorzugsweise kann er auch in einem Bereich von 110° bis 130° liegen.

Gemäß einer weiteren bevorzugten Ausführungsform der Erfindung kann vorgesehen sein, dass das Zielgerät eine zweite Verbindungseinrichtung aufweist, welche mit der ersten Verbindungseinrichtung in einer Verbindungsstellung mit den mindestens zwei Implantaten des Implantatsets verbindbar ist. Auf diese Weise kann das Zielgerät einfach und sicher mit den mindestens zwei Implantaten verbunden werden.

Sowohl Handhabung als auch Montage des Zielgeräts am Implantat werden weiter vereinfacht, wenn das Zielgerät einen ersten Endbereich und einen zweiten Endbereich aufweist und wenn die zweite Verbindungseinrichtung am ersten Endbereich vorgesehen ist. Beispielsweise kann so das Zielgerät ein proximales Ende eines oder mehrerer der Implantate des Implantatsets verlängern. So kann das Implantatset insbesondere auch für minimalinvasive chirurgische Eingriffe verwendet werden.

Herstellung und Aufbau des Implantatsets vereinfachen sich weiter, wenn das Zielgerät im Wesentlichen U-förmig ausgebildet ist mit einem ersten, den ersten Endbereich umfassenden Schenkel, einem zweiten, den zweiten Endbereich umfassenden Schenkel und einem die Schenkel verbindenden Verbindungsabschnitt.

Das Einsetzen eines Implantats des Implantatsets wird besonders einfach, wenn der erste und der zweite Schenkel parallel oder im Wesentlichen parallel zueinander ausgerichtet sind. So kann ein Operateur ohne Weiteres aus einer Orientierung des ersten Schenkels auf eine Orientierung des Implantats schlie-ßen, auch wenn dieses bereits in den Körper eines Patienten eingeführt ist.

Günstig ist es, wenn das Zielgerät mindestens eine Führung zum Führen eines Bohr- und/oder Fräswerkzeugs umfasst. So können an einem Knochen des Patienten gezielt Durchbrechungen, insbesondere Bohrungen, ausgebildet werden, und zwar ausgerichtet mit am Implantat vorgesehenen Befestigungselementaufnahmen, insbesondere in Form von Bohrungen und/oder Gewindebohrungen und/oder Langlöchern.

Um einen Abstand zwischen der Führung und einer Befestigungselementaufnahme am Implantat möglichst gering zu halten, ist es vorteilhaft, wenn die Führung am zweiten Endbereich vorgesehen ist. Sie kann dann insbesondere direkt einer Befestigungselementaufnahme eines Implantats gegenüberliegend vorgesehen werden. Ein kurzer Abstand zwischen der Führung und der Befestigungselementaufnahme verhindert eine Verformung eines Bearbeitungswerkzeugs entlang seiner Länge.

Besonders einfach wird der Aufbau des Zielgeräts, wenn die Führung mindestens eine Durchbrechung umfasst. Insbesondere können genauso viele Durchbrechungen am Zielgerät vorgesehen werden wie Befestigungselementaufnahmen am Implantat ausgebildet sind. So lässt sich insbesondere jeder Befestigungselementaufnahme eine eigene Durchbrechung zuordnen. Abdeckungen für nicht benötigte Durchbrechungen sind somit nicht erforderlich.

Um eine unerwünschte Beschädigung des Knochens, in oder an welchem das Implantat festgelegt werden soll, zu vermeiden, ist es günstig, wenn die mindestens eine Durchbrechung in der Verbindungsstellung, in welcher das Zielgerät mit einem Implantat verbunden ist, mit der mindestens einen Befestigungselementaufnahme des Implantats fluchtend ausgerichtet ist. Insbesondere kann die Durchbrechung fluchtend mit der mindestens einen Bohrung und/oder der mindestens einen Gewindebohrung ausgerichtet sein. Dies erlaubt es, Bearbeitungswerkzeuge mit minimalen Durchmessern zu verwenden, da so sichergestellt werden kann, dass freie Querschnitte einer mit einem Bearbeitungswerkzeug am Knochen ausgebildeten Durchbrechung und die Befestigungselementaufnahme am Implantat deckungsgleich ausgebildet werden.

Noch genauer kann ein Operateur eine Bohrung am Knochen ausbilden, um ein Befestigungselement durch eine am Implantat vorgesehene Befestigungselementaufnahme durchführen zu können, wenn eine von der mindestens einen Durchbrechung definierte Durchbrechungslängsachse in der Verbindungsstellung eine Bohrungslängsachse der mindestens einen Bohrung und/oder der mindestens einen Gewindebohrung definiert.

Besonders einfach herzustellen wird das Zielgerät, wenn die mindestens eine Durchbrechung in Form einer Zielbohrung ausgebildet ist.

Verletzungen von Körpergewebe des Patienten lassen sich einfach und sicher vermeiden, wenn die Führung mindestens eine Hülse für ein Bohr- und/oder Fräswerkzeug umfasst. Die Hülse kann insbesondere derart geformt sein, dass sie bis an den zu bearbeitenden Knochen des Patienten heranreicht. Insbesondere kann durch die Hülse auch von einem Knochen abgetragenes Material aus dem Körper des Patienten geleitet werden.

Besonders einfach in der Herstellung und im Aufbau wird das Zielgerät, wenn die mindestens eine Hülse in der mindestens einen Durchbrechung geführt und/oder gehalten ist.

Besonders gut reinigen lässt sich das Implantatset, insbesondere das Zielgerät, wenn die mindestens eine Hülse mit dem Zielgerät lösbar verbindbar ist.

Vorteilhafterweise ist die mindestens eine Hülse am Zielgerät verschiebbar gelagert. So kann sie individuell vom Zielgerät bis an einen zu bearbeitenden Knochen des Patienten herangeführt werden. Dies ist insbesondere dann günstig, wenn unterschiedlich gekrümmte Implantate vorgesehen sind, so dass ein Abstand zwischen der Führung des Zielgeräts und dem Implantat stark variieren kann.

Der Aufbau des Implantatsets wird besonders einfach, wenn nur ein einziges Zielgerät vorgesehen ist, welches mit allen Implantaten des Implantatsets direkt und ohne die Verwendung von Zwischenstücken, Aufsätzen und/oder Adaptern verbindbar ist. So kann ein Operateur das Zielgerät einfach und sicher direkt mit einem Implantat des Implantatsets verbinden, ohne darauf achten zu müssen, mit welchem Implantat er das Zielgerät verbinden möchte.

Gemäß einer bevorzugten Ausführungsform der Erfindung kann vorgesehen sein, dass die zweite Verbindungseinrichtung korrespondierend zur ersten Verbindungseinrichtung ausgebildet ist und dass die erste und zweite Verbindungseinrichtung in der Verbindungsstellung kraft- und/oder formschlüssig miteinander verbunden sind. So kann eine stabile und sichere Verbindung zwischen dem Zielgerät und dem Implantat hergestellt werden.

Der Aufbau des Implantatsets vereinfacht sich weiter, wenn die zweite Verbindungseinrichtung mindestens ein zweites Verbindungselement umfasst, welches mit dem ersten Verbindungselement in der Verbindungsstellung kraft- und/oder formschlüssig in Eingriff steht. Insbesondere kann so mit nur zwei Verbindungselementen eine sichere Verbindung zwischen dem Zielgerät und dem Implantat hergestellt werden.

Um das Zielgerät einfach vom Implantat lösen zu können, auch wenn das Implantat bereits im Körper des Patienten festgelegt ist, ist es günstig, wenn das erste oder das zweite Verbindungselement einen Außengewindeabschnitt umfasst und wenn das korrespondierende andere Verbindungselement einen zum Außengewindeabschnitt korrespondierenden Innengewindeabschnitt umfasst. So kann das Zielgerät durch Verschrauben mit dem Implantat verbunden werden.

Eine besonders einfache Verbindung lässt sich herstellen, wenn das erste oder das zweite Verbindungselement in Form eines Schraubbolzens ausgebildet ist, welcher einen langgestreckten Kopfteil aufweist, an welchem der Außengewindeschaft angeordnet ist. Vorzugsweise ist der Schraubbolzen am Zielgerät beweglich gehalten, so dass eine Verbindung beziehungsweise ein Lösen des Zielgeräts vom Implantat allein durch eine Verdrehung des Schraubbolzens erreicht werden kann.

Günstig ist es, wenn das Zielgerät eine Verbindungselementführung zum Führen und/oder Lagern des zweiten Verbindungselements umfasst. Dadurch lässt sich eine Stabilität einer Verbindung des Zielgeräts mit dem Implantat in der Verbindungsstellung deutlich erhöhen, denn beispielsweise kann sich das zweite Verbindungselement in der Verbindungselementführung abstützen.

Um eine möglichst schlanke und vorzugsweise für minimal-invasive chirurgische Eingriffe optimierte Bauform des Zielgeräts zu ermöglichen, ist es vorteilhaft, wenn die Verbindungselementführung koaxial oder im Wesentlichen koaxial zu einer vom zweiten Endbereich definierten Längsachse ausgebildet ist. So lässt sich insbesondere auch ein Gewicht des Zielgeräts minimieren.

Vorteilhafterweise weist die Verbindungselementführung mindestens einen Anschlag auf, an welchem sich ein Teil des zweiten Verbindungselements in der Verbindungsstellung abstützt. So kann sichergestellt werden, dass das zweite Verbindungselement sich in der Verbindungsstellung nicht vom Zielgerät lösen kann.

Vorzugsweise sind das Zielgerät und ein mit ihm verbundenes Implantat in einer Verbindungsstellung kraft- und/oder formschlüssig aneinander gehalten. So kann eine ausreichende Stabilität der Gesamtanordnung aus Zielgerät und Implantat sichergestellt werden, um ein Bearbeitungswerkzeug mit dem Zielgerät in gewünschter Weise führen zu können.

Besonders einfach und sicher lassen sich das Zielgerät und ein Implantat miteinander verbinden, wenn das Zielgerät und eines der mindestens zwei Implantate in der Verbindungsstellung klemmend aneinander gehalten sind.

Grundsätzlich wäre es denkbar, das Implantatset in unterschiedlichen Formen und für unterschiedliche Anwendungen auszubilden. Günstig ist es, wenn die Implantate des Implantatsets Marknägel sind. Marknägel können in einen Markraum eines Knochens eingesetzt werden, ohne den Knochen zusätzlich bearbeiten oder schwächen zu müssen. Denkbar sind insbesondere Ausgestaltungen in Form von proximalen Femurnägeln, retrograden Femurnägeln, Nägeln für die Tibia oder Humerus und Radius.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Darstellung eines Implantatsets umfassend ein Zielgerät und ein erstes Implantat;
- Figur 2:: eine Schnittansicht längs Linie 2-2 in Figur 1;
- Figur 2a:: eine vergrößerte Teilansicht aus Figur 2 im Verbindungsbereich zwischen Zielgerät und erstem Implantat;
- Figur 3:: eine Seitenansicht des ersten Implantats im Verbindungsbereich zum Zielgerät;
- Figur 4:: eine Ansicht ähnlich Figur 3 eines zweiten Implantats;
- Figur 5:: eine Ansicht ähnlich Figur 3 eines dritten Implantats; und
- Figur 6:: eine perspektivische Ansicht ähnlich Figur 1 mit einem vierten Implantat des Implantatsets.

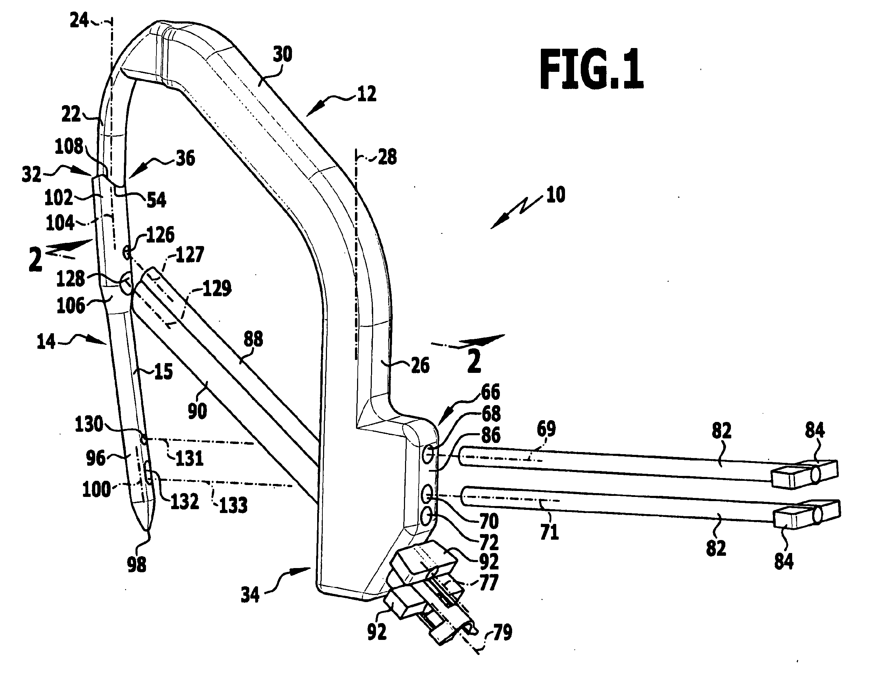

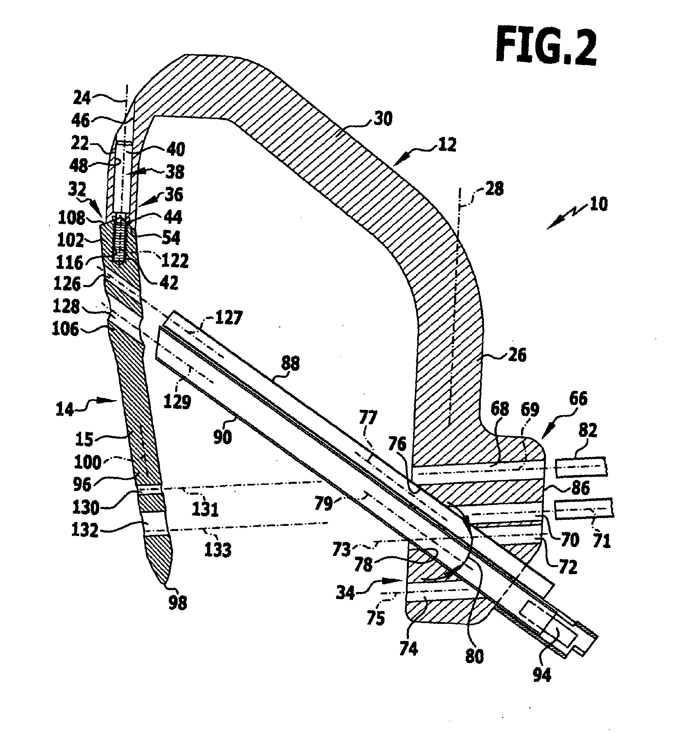

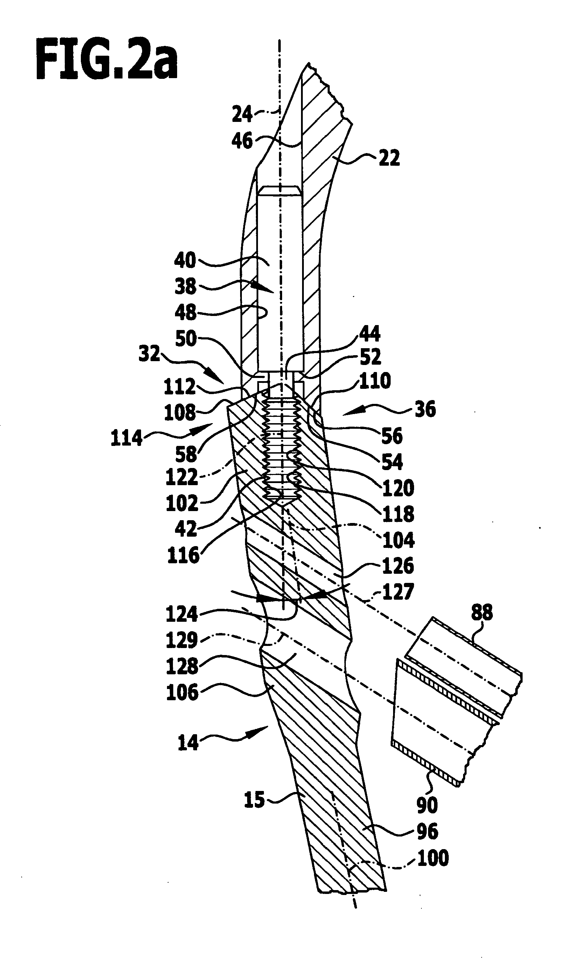

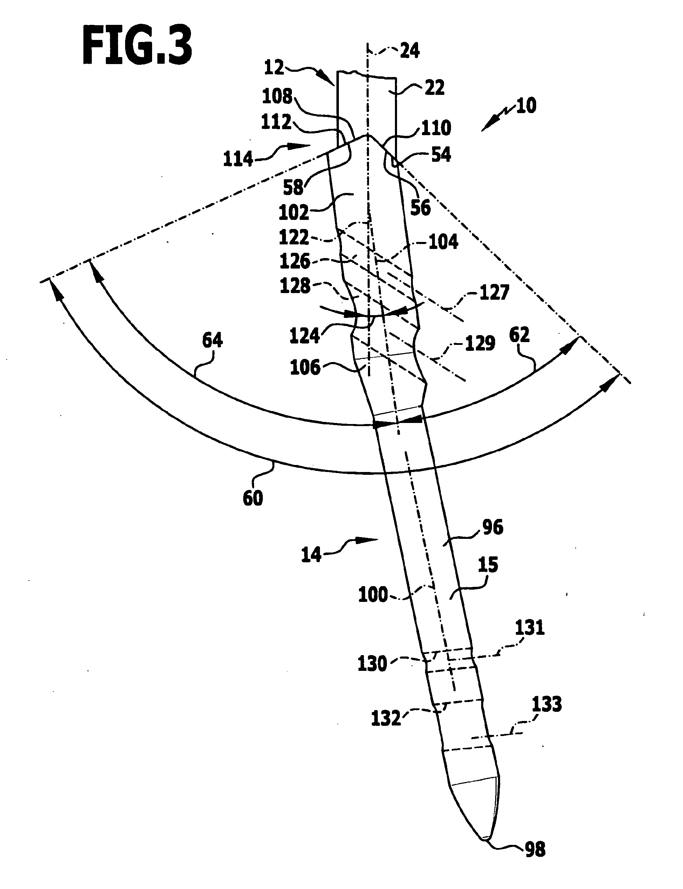

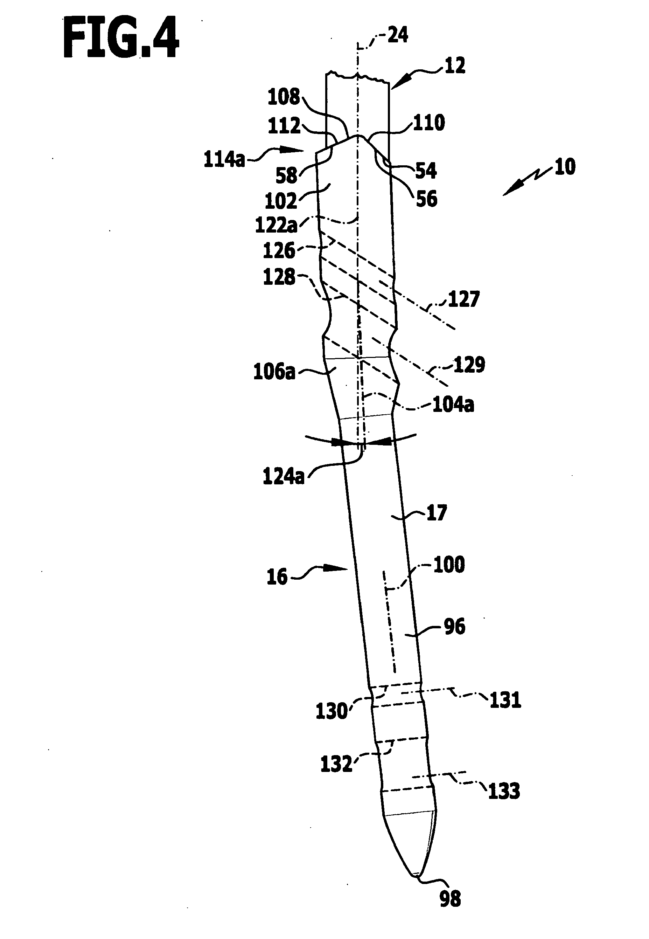

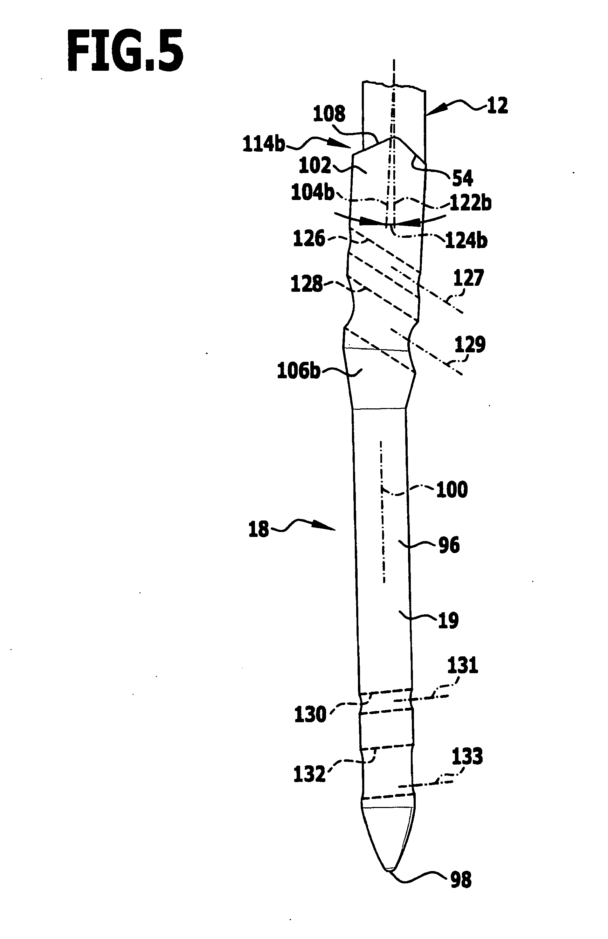

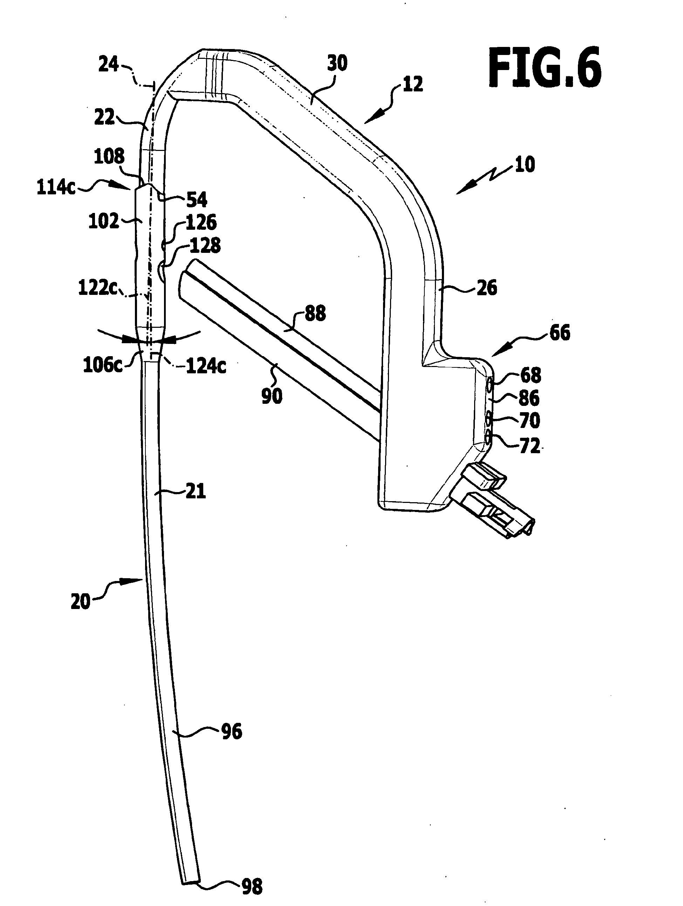

In den Figuren 1 bis 6 ist ein insgesamt mit dem Bezugszeichen 10 versehenes Implantatset dargestellt, welches ein Zielgerät 12 sowie mehrere Implantate, insbesondere die in den Figuren 1 bis 6 dargestellten Implantate 14, 16, 18 und 20, welche in Form von Marknägeln umfassend jeweils einen langgestreckten Schaft 15, 17, 19, 21 ausgebildet sind, umfasst. Die Implantate 14, 16, 18 und 20 können insbesondere zur Behandlung von Brüchen oder zur Stabilisierung von Röhrenknochen verwendet werden, beispielsweise einer Tibia, eines Femurs oder eines Humerus eines menschlichen oder tierischen Körpers.

Das Zielgerät 12 ist im Wesentlichen U-förmig ausgebildet und weist einen ersten Schenkel 22, welcher eine Längsachse 24 definiert, und einen zweiten Schenkel 26 auf, dessen Längsachse 28 parallel zur Längsachse 24 verläuft und der etwa doppelt so lang ist wie der Schenkel 22. Ein etwa unter einem Winkel von 45° relativ zu den beiden Längsachsen 24 und 28 orientierter Verbindungsabschnitt 30 verbindet die beiden Schenkel 22 und 26 miteinander. Am ersten Schenkel 22 ist ein erster Endbereich des Zielgeräts ausgebildet, am zweiten Schenkel 26, insbesondere an dessen freiem Ende, ein zweiter Endbereich 34.

Am ersten Endbereich 32 ist eine im Sinne der Ansprüche zweite Verbindungseinrichtung 36 vorgesehen. Sie umfasst ein zweites Verbindungselement 38 in Form eines Schraubbolzens, welcher einen langgestreckten zylindrischen Kopfteil 40 aufweist und einen im Außendurchmesser verringerten, koaxial vom Kopfteil 40 abstehenden, einen Außengewindeabschnitt 42 umfassenden zylindrischen Schaftteil 44. Das zweite Verbindungselement 38 ist in einer Verbindungselementführung 46 in Form einer Bohrung 48 gehalten, deren Längsachse koaxial zur Längsachse 24 ausgerichtet ist. Benachbart eines distalen Endes der Bohrung 48 ist in dieser ein ringförmiger, auf die Längsachse 24 hin weisender, einen Anschlag 50 bildender Vorsprung vorgesehen, der einen Innendurchmesser aufweist, welcher es ermöglicht, dass der Außengewindeabschnitt 42, durch ihn hindurchgesteckt werden kann, jedoch eine ringförmige Anschlagfläche 52, die am Kopfteil 40 ausgebildet ist, den Schaftteil 44 umgibt und in distaler Richtung weist, am Anschlag 50 anliegen kann. So wird eine Bewegung des zweiten Verbindungselements 38 in distaler Richtung aus der Verbindungselementführung 46 heraus verhindert.

Ein distales Ende der Bohrung 48 umgebend ist ein eine Kontur bildender Rand 54 in zwei Randbereiche 56 und 58 geteilt, die relativ zueinander um einen Winkel 60 von etwa 110° geneigt sind. Der Randbereich 56 ist etwa unter einem Winkel 62 von 45° relativ zur Längsachse 24 geneigt, der Randbereich 58 unter einem Winkel 64 von etwa 65°.

Der zweite Endbereich 34 des Zielgeräts 12 ist im Wesentlichen quaderförmig ausgebildet und etwa doppelt so breit wie der zweite Schenkel 26 in einem Bereich, welcher sich direkt an den Verbindungsabschnitt 30 anschließt. Am zweiten Endbereich 34 ist eine Führung 66 ausgebildet, die zum Führen eines in den Figuren nicht näher dargestellten Bohr- und/oder Fräswerkzeugs zur Knochenbearbeitung dient. Die Führung 66 umfasst vier Bohrungen 68, 70, 72 und 74, deren Längsachsen 69, 71, 73 und 75 parallel zueinander ausgerichtet und relativ zur Längsachse 28, die sie zudem schneiden, um einen Winkel von etwa 10° und vom Verbindungsabschnitt weg weisend geneigt sind. Die Längsachsen 69, 71, 73 und 75 liegen zudem alle in einer Ebene. Des Weiteren sind am zweiten Endbereich 34 zwei weitere Bohrungen 76 und 78 ausgebildet, deren Längsachsen 77 und 79 parallel zueinander verlaufen und in derselben Ebene liegen wie die Längsachsen 69, 71, 73 und 75. Allerdings schneiden die Längsachsen 77 und 79 die Längsachsen 69, 71, 73 und 75 unter einem Winkel 80 von etwa 40°. Zudem weisen die Längsachsen 77 und 79 in etwa in Richtung auf den ersten Endbereich 32 hin und verlaufen näherungsweise parallel zum Verbindungsabschnitt 30.

Ferner umfasst die Führung 66 zwei Hülsen 82, deren Außendurchmesser an einen Innendurchmesser der Bohrungen 68, 70, 72 und 74 angepasst ist, die alle denselben Innendurchmesser aufweisen. Die Hülse 82 weist an einem Ende einen quaderförmigen Anschlagkörper 84 auf, welcher bei maximal weit in eine der Bohrungen 68, 70, 72 oder 74 eingeschobener Hülse 82 an einer vom ersten Schenkel 22 weg weisenden Seitenfläche 86 des zweiten Endbereichs 34, die parallel zur Längsachse 28 verläuft, anschlägt. Die Hülse 82 dient dazu, beim Einführen eines nicht dargestellten, vorzugsweise rotierenden Bearbeitungswerkzeugs umliegendes Gewebe, welches sonst direkt mit dem Bearbeitungswerkzeug in Kontakt gelangen könnte, vor Verletzungen zu schützen.

Ferner umfasst die Führung 66 zwei weitere Hülsen 88 und 90, welche korrespondierend zu den Bohrungen 76 und 78 ausgebildet und in diesen verschiebbar gelagert sind. Die Hülsen 88 und 90 weisen ebenfalls an ihrem einen Ende einen quaderförmigen Anschlagkörper 92 beziehungsweise 94 auf, welche eine Einführtiefe der Hülsen 88 und 90 in den Bohrungen 76 und 78 begrenzen.

Das Zielgerät 12 kann beispielsweise mit dem in den Figuren 1 bis 3 dargestellten Implantat 14 verbunden werden, welches einen stabförmigen distalen Endabschnitt 96 aufweist, welcher sich etwa über zwei Drittel einer Gesamtlänge des Implantats 14 erstreckt. An einem distalen Ende ist der Endabschnitt 96 in Form einer stumpfen Spitze 98 ausgebildet. Er definiert ferner eine Längsachse 100. Ein proximaler Endbereich 102 des Implantats 14 ist ebenfalls in Form eines zylindrischen Stabes geformt, jedoch mit einem etwas größeren Außendurchmesser als der Endabschnitt 96. Der Endbereich 102 definiert eine Längsachse 104, welche relativ zur Längsachse 100 etwas geneigt ist, und zwar um einen Winkel von etwa 5°. Diese Abwinkelung wird realisiert durch einen im Verhältnis zur Gesamtlänge des Implantats 12 kurzen Krümmungsabschnitt 106, welcher den Endbereich 102 und den Endabschnitt 96 miteinander verbindet.

Eine proximale Endfläche 108 des Endbereichs 102, welche eine in proximaler Richtung weisende Kontur definiert, umfasst zwei gegeneinander um den Winkel 60 geneigte Flächenbereiche 110 und 112. Am Endbereich 102 ist eine im Sinne der Ansprüche erste Verbindungseinrichtung 114 vorgesehen. Sie umfasst die beiden Flächenbereiche 110 und 112 sowie ein im Sinne der Ansprüche erstes Verbindungselement 116 in Form eines mit einem Innengewinde 118 versehenen Sacklochs 120. Eine Längsachse 122 des Sacklochs 120 definiert eine Verbindungsrichtung. Sie ist relativ zur Längsachse 104 um einen Winkel 124 geneigt, der bei dem in den Figuren 1 bis 3 dargestellten Ausführungsbeispiel einen Wert von circa 12° aufweist.

Des Weiteren sind am Endbereich 102 zwei Bohrungen 126 und 128 vorgesehen, wobei ein Innendurchmesser der Bohrung 128, die weiter vom Sackloch 120 entfernt ist als die Bohrung 126, etwa doppelt so groß ist als ein Innendurchmesser der Bohrung 126. Die Bohrungen 126 und 128 definieren Längsachsen 127 beziehungsweise 129.

Im Bereich eines distalen Endes des Endabschnitts 96 ist eine weitere Bohrung 130 mit einer Längsachse 131 ausgebildet, deren Innendurchmesser in etwa dem Innendurchmesser der Bohrung 126 entspricht. Zwischen der Bohrung 126 und der Spitze 98 ist ein Langloch 132 ausgebildet, welches eine Durchbrechungsrichtung 133 definiert, welche parallel zur Längsachse 131 verläuft.

Das Implantat 14 kann mit dem Zielgerät 12 auf einfache Weise verbunden werden. Hierzu wird der Außengewindeabschnitt 42 des zweiten Verbindungselements 38 in das korrespondierende Innengewinde 118 des Sacklochs 120 eingeschraubt, und zwar so weit, bis der Flächenbereich 112 am Randbereich 58, der Flächenbereich 110 am Randbereich 56 und die Anschlagfläche 52 am Anschlag 50 anliegen. Das Zielgerät 12 ist dann kraft- und formschlüssig mit dem Implantat 14 über die Schraubverbindung verbunden und nimmt die in den Figuren 1 bis 3 dargestellte Verbindungsstellung ein.

In der Verbindungsstellung fluchten die Bohrungen 68 und 70 mit den Bohrungen 130 und 132, insbesondere fallen die Längsachsen 69 und 71 mit den Längsachsen 131 und 133 zusammen. In analoger Weise fluchten die Bohrungen 76 und 78 mit den Bohrungen 126 und 128. Die Längsachsen 77 und 79 fallen ebenfalls mit den Längsachsen 127 und 129 zusammen. So kann ein Operateur beispielsweise die beiden Hülsen 82 in die Bohrungen 68 und 70 schieben und nach Eröffnen des Körpers des Patienten durch zwei kleine Inzisionen vordere Enden der Hülsen 82 in den Körper ein- und bis an den Knochen heranführen, in den das Implantat 14 eingesetzt ist. Mit Fräs- und/oder Bohrwerkzeugen kann dann der Knochen eröffnet werden, und zwar bis an das Implantat 14 heran. Die Hülsen 82 verhindern, dass umliegendes Gewebe durch das Bearbeitungswerkzeug verletzt wird. Das Implantat 14 kann mit nicht dargestellten Befestigungselementen, beispielsweise Knochenschrauben oder Nägeln, am Knochen festgelegt werden, und zwar dadurch, dass die den Knochen und die jeweilige Bohrung 130 beziehungsweise 132 durchsetzenden Befestigungselemente am Knochen festgelegt werden.

In analoger Weise können die Hülsen 88 und 90 in die Bohrungen 76 und 78 des Zielgeräts 12 eingeführt und bis an den Knochen des Patienten herangeführt werden. Auch hier sind nur eine oder zwei kleine Inzisionen erforderlich, um die Hülsen 88 und 90 in den Körper des Patienten ein- und an den Knochen heranzuführen. Mit Befestigungselementen, die die Bohrungen 126 und 128 durchsetzen, kann auch der proximale Endbereich 102 des Implantats am Knochen des Patienten festgelegt werden.

Das Implantatset 10 ist so gestaltet, dass nur ein einziges, nämlich das Zielgerät 12 benötigt wird und keine weiteren Zielgeräte. Dies wird dadurch realisiert, dass die sich vom Implantat 14 unterscheidenden Implantate 16, 18 und 20 jeweils eine erste Verbindungseinrichtung 114a, 114b und 114c aufweisen, die eine unterschiedliche Verbindungsrichtung definieren. Dies wird dadurch erreicht, dass in den jeweiligen proximalen Endbereich 102 das erste Verbindungselement 116 unter einem anderen Winkel 124a, 124b beziehungsweise 124c relativ zur Längsachse 104a, 104b beziehungsweise 104c orientiert ist als die Längsachse 104. Folglich unterscheiden sich die Implantate 14, 16, 18 und 20 im jeweiligen Winkel 124, 124a, 124b und 124c.

Die ersten Verbindungseinrichtungen 114, 114a, 114b und 114c bezogen auf die jeweilige, durch den proximalen Endbereich 102 definierte Längsachse 104, 104a, 104b und 104c definierten somit jeweils eine andere Verbindungsrichtung zum Verbinden des jeweiligen Implantats 14, 16, 18 und 20 mit dem Zielgerät 12. Der Winkel 124a weist einen Wert von etwa 7° auf, der Winkel 124b einen Wert von etwa 2°. Somit unterscheiden sich die zwischen den vom ersten Verbindungselement 116 definierten Längsachsen 24 der Implantate 14, 16, 18 und 20 und den durch die jeweiligen proximalen Endbereiche 102 definierten Längsachsen 104, 104a und 104b definierten Winkel 124, 124a und 124b um jeweils einen Winkel von 5°. Diese Winkeldifferenz könnte zwischen den einzelnen Implantaten auch unterschiedlich ausfallen. Vorzugsweise liegt diese Winkeldifferenz in einem Bereich von 3° bis 8°. Zwar definieren die Implantate 14, 16, 18 und 20 unterschiedliche Verbindungsrichtungen, doch fallen diese auf Grund der Ausgestaltung der ersten Verbindungseinrichtung 114 und der zweiten Verbindungseinrichtung 36 mit der Längsachse 24 zusammen.

Da die Implantate 14, 16, 18 und 20 sich in ihrer Länge oder in der unterschiedlichen Gestaltung des Krümmungsabschnitts 106 unterscheiden, ermöglicht es die jeweils entsprechend ausgebildete erste Verbindungseinrichtung 114, 114a, 114b und 114c, dass die Längsachsen 69, 71, 77 und 79 koaxial zur jeweils korrespondierenden Längsachse 127, 129, 131 und 133 der Bohrungen 126, 128, 130 und 132 an den Implantaten 14, 16, 18 und 20 in der Verbindungsstellung ausgerichtet sind.

Bei dem oben beschriebenen Implantatset 10 sind somit keine Adapter, Zwischenstücke, Aufsätze oder irgendwelche Abdeckvorrichtungen zum Abdecken bestimmter Bohrungen am Zielgerät 12 erforderlich, um mit nur einem einzigen Zielgerät 12 unterschiedlich lange, unterschiedlich geformte, unterschiedlich gekrümmte oder unterschiedliche CCD-Winkel aufweisende Implantate mit dem Zielgerät 12 verbinden zu können. Das Zielgerät 12 wird stets direkt mit dem jeweiligen Implantat verbunden. Die Führung 66 am Zielgerät 12 ist somit immer optimal und passend zum jeweiligen Implantat 14, 16, 18 und 20 orientiert, um Bohrungen am Knochen des Patienten zum Einführen von Befestigungselementen zu setzen.

Das Zielgerät 12 ist vorzugsweise aus einem sterilisierbaren Werkstoff, aus Stabilitätsgründen insbesondere aus einem Instrumentenstahl oder einem faserverstärkten Kunststoff gefertigt. Die Implantate 14, 16, 18 und 20 sind aus einem körperverträglichen Material hergestellt und können beispielsweise aus einem Kunststoff oder aus einem Metall hergestellt sein.

## Patentansprüche

1. Implantatset (10) umfassend mindestens zwei unterschiedlich geformte Implantate (14, 16, 18, 20), welche jeweils einen Schaft (15, 17, 19, 21), welcher ein proximales und ein distales Ende aufweist, sowie eine am proximalen Ende vorgesehene erste Verbindungseinrichtung (114, 114a, 114b, 114c) zum Verbinden mit einem Zielgerät (12) umfassen, wobei die erste Verbindungseinrichtung (114, 114a, 114b, 114c) eine Verbindungsrichtung definiert, **dadurch gekennzeichnet, dass** mindestens zwei Implantate (14, 16, 18, 20) erste Verbindungseinrichtungen (114, 114a, 114b, 114c) aufweisen, welche bezogen auf eine durch einen proximalen Endbereich (102) des jeweiligen Schafts (15, 17, 19, 21) definierte Längsachse (104, 104a, 104b, 104c) unterschiedlich geneigte Verbindungsrichtungen definieren.

2. Implantatset nach Anspruch 1, **dadurch gekennzeichnet, dass** Verbindungsrichtungen unterschiedlicher Implantate (14, 16, 18, 20) relativ zu den durch die jeweiligen proximalen Endbereiche (102) definierten Längsachsen (104, 104a, 104b, 104c) um einen Winkel (124, 124a, 124b, 124c) in einem Bereich von 0° bis 40° geneigt sind.

3. Implantatset nach Anspruch 1 oder 2, **dadurch gekennzeichnet, dass** sich Verbindungsrichtungen unterschiedlicher Implantate (14, 16, 18, 20) relativ zu den durch die jeweiligen proximalen Endbereiche (102) definierten Längsachsen um jeweils einen Winkel (124, 124a, 124b, 124c) in einem Bereich von 3° bis 8° unterscheiden.

4. Implantatset nach einem der voranstehenden Ansprüche, **dadurch gekennzeichnet, dass** die Schäfte (15, 17, 19, 21) der mindestens zwei unterschiedlich geformten Implantate (14, 16, 18, 20) unterschiedlich lang sind.

5. Implantatset nach einem der voranstehenden Ansprüche, **dadurch gekennzeichnet, dass** die Schäfte (15, 17, 19, 21) der mindestens zwei unterschiedlich geformten Implantate (14, 16, 18, 20) unterschiedlich gekrümmt sind.

6. Implantatset nach einem der voranstehenden Ansprüche, **dadurch gekennzeichnet, dass** die Schäfte (15, 17, 19, 21) der mindestens zwei unterschiedlich geformten Implantate (14, 16, 18, 20) unterschiedliche CCD-Winkel (Centrum-Collum-Diaphysen-Winkel) definieren.

7. Implantatset nach einem der voranstehenden Ansprüche, **dadurch gekennzeichnet, dass** die mindestens zwei Implantate (14, 16, 18, 20) jeweils mindestens eine Befestigungselementaufnahme (126, 128, 130, 132) aufweisen.

8. Implantatset nach Anspruch 7, **dadurch gekennzeichnet, dass** mindestens zwei Befestigungselementaufnahmen (126, 128, 130, 132) vorgesehen sind, welche relativ zueinander bei den mindestens zwei unterschiedlich geformten Implantaten (14, 16, 18, 20) identisch ausgerichtet sind.

9. Implantatset nach Anspruch 7 oder 8, **dadurch gekennzeichnet, dass** die mindestens eine Befestigungselementaufnahme (126, 128, 130, 132) an einem proximalen und/oder einem distalen Endbereich (102) des Schafts (15, 17, 19, 21) vorgesehen ist.

10. Implantatset nach einem der voranstehenden Ansprüche, **dadurch gekennzeichnet, dass** die erste Verbindungseinrichtung (114, 114a, 114b, 114c) mindestens ein erstes Verbindungselement (116) umfasst.

11. Implantatset nach Anspruch 10, **dadurch gekennzeichnet, dass** das mindestens eine erste Verbindungselement (116) ein mit einem Innengewinde (118) versehenes Sackloch (120) umfasst.

12. Implantatset nach Anspruch 10 oder 11, **dadurch gekennzeichnet, dass** eine vom ersten Verbindungselement (116) definierte Längsachse (24) relativ zu einer durch den proximalen Endbereich (102) des jeweiligen Schafts (15, 17, 19, 21) definierte Längsachse (104, 104a, 104b, 104c) bei mindestens zwei Implantaten (14, 16, 18, 20) unterschiedlich geneigt ist.

13. Implantatset nach Anspruch 12, **dadurch gekennzeichnet, dass** die von den ersten Verbindungselementen (116) der Implantate (14, 16, 18, 20) definierten Längsachsen (24) relativ zu den durch die jeweiligen proximalen Endbereiche (102) definierten Längsachsen (104, 104a, 104b, 104c) um einen Winkel (124, 124a, 124b, 124c) in einem Bereich von 0° bis 40° geneigt sind.

14. Implantatset nach Anspruch 12 oder 13, **dadurch gekennzeichnet, dass** sich die von den ersten Verbindungselementen (116) definierten Längsachsen (24) unterschiedlicher Implantate (14, 16, 18, 20) relativ zu den durch die jeweiligen proximalen Endbereiche (102) definierten Längsachsen (104, 104a, 104b, 104c) um jeweils einen Winkel (124, 124a, 124b, 124c) in einem Bereich von 3° bis 8° unterscheiden.

15. Implantatset nach einem der voranstehenden Ansprüche, **dadurch gekennzeichnet, dass** eine in proximaler Richtung weisende Kontur (108) des Schafts (15, 17, 19, 21) ausgebildet ist, welche Kontur (108) zwei relativ zueinander um einen Neigungswinkel (60) geneigte Flächenbereiche (110, 112) aufweist.

16. Implantatset nach Anspruch 15, **dadurch gekennzeichnet, dass** der Neigungswinkel (60) einen Wert in einem Bereich von 90° bis 150° aufweist.

17. Implantatset nach einem der voranstehenden Ansprüche, **gekennzeichnet durch** ein Zielgerät (12) mit einer zweiten Verbindungseinrichtung (36), welche mit der ersten Verbindungseinrichtung (114) in einer Verbindungsstellung mit den mindestens zwei Implantaten (14, 16, 18, 20) des Implantatsets (10) verbindbar ist.

18. Implantatset nach Anspruch 17, **dadurch gekennzeichnet, dass** eine in proximaler Richtung weisende Kontur (108) des Schafts (15, 17, 19, 21) korrespondierend zu einem am Zielgerät (12) vorgesehenen Anlagebereich (54) ausgebildet ist.

19. Implantatset nach Anspruch 17 oder 18, **dadurch gekennzeichnet, dass** das Zielgerät (12) einen ersten Endbereich (32) und einen zweiten Endbereich (34) aufweist und dass die zweite Verbindungseinrichtung (36) am ersten Endbereich (32) vorgesehen ist.

20. Implantatset nach Anspruch 19, **dadurch gekennzeichnet, dass** das Zielgerät (12) im Wesentlichen U-förmig ausgebildet ist mit einem ersten, den ersten Endbereich (32) umfassenden Schenkel (22), einem zweiten, den zweiten Endbereich (34) umfassenden Schenkel (26) und einem die Schenkel (22, 26) verbindenden Verbindungsabschnitt (30).

21. Implantatset nach Anspruch 20, **dadurch gekennzeichnet, dass** der erste und der zweite Schenkel (22, 26) parallel oder im Wesentlichen parallel zueinander ausgerichtet sind.

22. Implantatset nach einem der Ansprüche 17 bis 21, **dadurch gekennzeichnet, dass** das Zielgerät (12) mindestens eine Führung (66) zum Führen eines Bohr- und/oder Fräswerkzeugs umfasst.

23. Implantatset nach Anspruch 22, **dadurch gekennzeichnet, dass** die Führung (66) am zweiten Endbereich (34) vorgesehen ist.

24. Implantatset nach Anspruch 22 oder 23, **dadurch gekennzeichnet, dass** die Führung (66) mindestens eine Durchbrechung (68, 70, 72, 74) umfasst.

25. Implantatset nach Anspruch 24, **dadurch gekennzeichnet, dass** die mindestens eine Durchbrechung (68, 70, 72, 74) in der Verbindungsstellung, in welcher das Zielgerät (12) mit einem Implantat (14, 16, 18, 20) verbunden ist, mit der mindestens einen Bohrung (126, 128, 130, 132) und/oder der mindestens einen Gewindebohrung des Implantats (14, 16, 18, 20) fluchtend ausgerichtet ist.

26. Implantatset nach Anspruch 24 oder 25, **dadurch gekennzeichnet, dass** eine von der mindestens einen Durchbrechung (68, 70, 72, 74) definierte Durchbrechungslängsachse (69, 71, 73, 75) in der Verbindungsstellung eine Bohrungslängsachse (127, 129, 131, 133) der mindestens einen Bohrung (126, 128, 130, 132) und/oder der mindestens einen Gewindebohrung definiert.

27. Implantatset nach einem der Ansprüche 24 bis 26, **dadurch gekennzeichnet, dass** die mindestens eine Durchbrechung (68, 70, 72, 74) in Form einer Zielbohrung (68, 70, 72, 74) ausgebildet ist.

28. Implantatset nach einem der Ansprüche 24 bis 27, **dadurch gekennzeichnet, dass** die Führung (66) mindestens eine Hülse (82, 88, 90) für ein Bohr- und/oder Fräswerkzeug umfasst.

29. Implantatset nach Anspruch 28, **dadurch gekennzeichnet, dass** die mindestens eine Hülse (82, 88, 90) in der mindestens einen Durchbrechung (68, 70, 72, 74) geführt und/oder gehalten ist.

30. Implantatset nach Anspruch 28 oder 29, **dadurch gekennzeichnet, dass** die mindestens eine Hülse (82, 88, 90) mit dem Zielgerät (12) lösbar verbindbar ist.

31. Implantatset nach einem der Ansprüche 28 bis 30, **dadurch gekennzeichnet, dass** die mindestens eine Hülse (82, 88, 90) am Zielgerät (12) verschiebbar gelagert ist.

32. Implantatset nach einem der Ansprüche 17 bis 31, **dadurch gekennzeichnet, dass** nur ein einziges Zielgerät (12) vorgesehen ist, welches mit allen Implantaten (14, 16, 18, 20) des Implantatsets (10) direkt und ohne die Verwendung von Zwischenstücken, Aufsätzen und/oder Adaptern verbindbar ist.

33. Implantatset nach einem der Ansprüche 17 bis 32, **dadurch gekennzeichnet, dass** die zweite Verbindungseinrichtung (36) korrespondierend zur ersten Verbindungseinrichtung (114) ausgebildet ist und dass die erste und zweite Verbindungseinrichtung (114, 36) in der Verbindungsstellung kraft- und/oder formschlüssig miteinander verbunden sind.

34. Implantatset nach einem der Ansprüche 17 bis 33, **dadurch gekennzeichnet, dass** die zweite Verbindungseinrichtung (36) mindestens ein zweites Verbindungselement (38) umfasst, welches mit dem ersten Verbindungselement (116) in der Verbindungsstellung kraft- und/oder formschlüssig in Eingriff steht.

35. Implantatset nach Anspruch 34, **dadurch gekennzeichnet, dass** das erste oder das zweite Verbindungselement (38) einen Außengewindeabschnitt (42) umfasst und dass das korrespondierende andere Verbindungselement (116) einen zum Außengewindeabschnitt (42), korrespondierenden Innengewindeabschnitt (118) umfasst.

36. Implantatset nach Anspruch 35, **dadurch gekennzeichnet, dass** das erste oder das zweite Verbindungselement (116) in Form eines Schraubbolzens (116) ausgebildet ist, welcher einen langgestreckten Kopfteil (40) aufweist, an welchem der Außengewindeabschnitt (42) angeordnet ist.

37. Implantatset nach einem der Ansprüche 34 bis 36, **dadurch gekennzeichnet, dass** das Zielgerät (12) eine Verbindungselementführung (46) zum Führen und/oder Lagern des zweiten Verbindungselements (38) umfasst.

38. Implantatset nach Anspruch 37, **dadurch gekennzeichnet, dass** die Verbindungselementführung (46) of koaxial oder im Wesentlichen koaxial zu einer vom ersten Endbereich (32) definierten Längsachse (24) ausgebildet ist.

39. Implantatset nach Anspruch 37 oder 38, **dadurch gekennzeichnet, dass** die Verbindungselementführung (46) mindestens einen Anschlag (50) aufweist, an welchem sich ein Teil (40) des zweiten Verbindungselements (38) in der Verbindungsstellung abstützt.

40. Implantatset nach einem der Ansprüche 17 bis 39, **dadurch gekennzeichnet, dass** das Zielgerät (12) und ein mit ihm verbundenes Implantat (14, 16, 18, 20) in einer Verbindungsstellung kraft- und/oder formschlüssig aneinander gehalten sind.

41. Implantatset nach Anspruch 40, **dadurch gekennzeichnet, dass** das Zielgerät (12) und eines der mindestens zwei Implantate (14, 16, 18, 20) in der Verbindungsstellung klemmend aneinander gehalten sind.

42. Implantatset nach einem der voranstehenden Ansprüche, **dadurch gekennzeichnet, dass** die Implantate (14, 16, 18, 20) des Implantatsets (10) Marknägel sind.

## Claims

1. Implant set (10) comprising at least two differently shaped implants (14, 16, 18, 20), each having a shaft (15, 17, 19, 21) with a proximal end and a distal end, and a first connecting device (114, 114a, 114b, 114c) provided at the proximal end for connection to a targeting device (12), the first connecting device (114, 114a, 114b, 114c) defining a connecting direction, **characterized in that** at least two implants (14, 16, 18, 20) comprise first connecting devices (114, 114a, 114b, 114c) which, relative to a longitudinal axis (104, 104a, 104b, 104 c) defined by a proximal end area (102) of the respective shaft (15, 17, 19, 21), define differently inclined connecting directions.

2. Implant set in accordance with claim 1, **characterized in that** connecting directions of different implants (14, 16, 18, 20) are inclined relative to the longitudinal axes (104, 104a, 104b, 104c) defined by the respective proximal end areas (102) at an angle (124, 124a, 124b, 124c) ranging from 0° to 40°.

3. Implant set in accordance with claim 1 or 2, **characterized in that** connecting directions of different implants (14, 16, 18, 20) differ relative to the longitudinal axes defined by the respective proximal end areas (102) by a respective angle (124, 124a, 124b, 124c) ranging from 3° to 8°.

4. Implant set in accordance with any one of the preceding claims, **characterized in that** the shafts (15, 17, 19, 21) of the at least two differently shaped implants (14, 16, 18, 20) are of different length.

5. Implant set in accordance with any one of the preceding claims, **characterized in that** the shafts (15, 17, 19, 21) of the at least two differently shaped implants (14, 16, 18, 20) have a different curvature.

6. Implant set in accordance with any one of the preceding claims, **characterized in that** the shafts (15, 17, 19, 21) of the at least two differently shaped implants (14, 16, 18, 20) define different CCD angles (caput-collum-diaphyseal angles).

7. Implant set in accordance with any one of the preceding claims, **characterized in that** the at least two implants (14, 16, 18, 20) each comprise at least one fixation element receptacle (126, 128, 130, 132).

8. Implant set in accordance with claim 7, **characterized in that** at least two fixation element receptacles (126, 128, 130, 132) are provided, which are identically aligned relative to each other in the at least two differently shaped implants (14, 16, 18, 20).

9. Implant set in accordance with claim 7 or 8, **characterized in that** the at least one fixation element receptacle (126, 128, 130, 132) is provided at a proximal and/or a distal end area (102) of the shaft (15, 17, 19, 21).

10. Implant set in accordance with any one of the preceding claims, **characterized in that** the first connecting device (114, 114a, 114b, 114c) comprises at least one first connecting element (116).

11. Implant set in accordance with claim 10, **characterized in that** the at least one first connecting element (116) comprises a blind hole (120) provided with an internal thread (118).

12. Implant set in accordance with claim 10 or 11, **characterized in that** a longitudinal axis (24) defined by the first connecting element (116) is differently inclined relative to a longitudinal axis (104, 104a, 104b, 104c) defined by the proximal end area (102) of the respective shaft (15, 17, 19, 21) in at least two implants (14, 16, 18, 20).

13. Implant set in accordance with claim 12, **characterized in that** the longitudinal axes (24) defined by the first connecting elements (116) of the implants (14, 16, 18, 20) are inclined relative to the longitudinal axes (104, 104a, 104b, 104c) defined by the respective proximal end areas (102) at an angle (124, 124a, 124b, 124c) ranging from 0° to 40°.

14. Implant set in accordance with claim 12 or 13, **characterized in that** the longitudinal axes (24), defined by the first connecting elements (116), of different implants (14, 16, 18, 20) differ relative to the longitudinal axes (104, 104a, 104b, 104c) defined by the respective proximal end areas (102) by a respective angle (124, 124a, 124b, 124c) ranging from 3° to 8°.

15. Implant set in accordance with any one of the preceding claims, **characterized in that** a contour (108), pointing in proximal direction, of the shaft (15, 17, 19, 21) is formed, which contour (108) comprises two surface areas (110, 112) inclined relative to each other at an angle of inclination (60).

16. Implant set in accordance with claim 15, **characterized in that** the angle of inclination (60) has a value ranging from 90° to 150°.

17. Implant set in accordance with any one of the preceding claims, **characterized by** a targeting device (12) with a second connecting device (36) which, in a connected position with the at least two implants (14, 16, 18, 20) of the implant set (10), is connectable to the first connecting device (114).

18. Implant set in accordance with claim 17, **characterized in that** a contour (108), pointing in proximal direction, of the shaft (15, 17, 19, 21) is formed so as to correspond to an abutment area (54) provided on the targeting device (12).

19. Implant set in accordance with claim 17 or 18, **characterized in that** the targeting device (12) comprises a first end area (32) and a second end area (34), and **in that** the second connecting device (36) is provided at the first end area (32).

20. Implant set in accordance with claim 19, **characterized in that** the targeting device (12) is of substantially U-shaped configuration with a first leg (22) comprising the first end area (32), a second leg (26) comprising the second end area (34), and a connecting portion (30) connecting the legs (22, 26).

21. Implant set in accordance with claim 20, **characterized in that** the first leg and the second leg (22, 26) are aligned parallel or substantially parallel to each other.

22. Implant set in accordance with any one of claims 17 to 21, **characterized in that** the targeting device (12) comprises at least one guide (66) for guiding a drilling and/or milling tool.

23. Implant set in accordance with claim 22, **characterized in that** the guide (66) is provided at the second end area (34).

24. Implant set in accordance with claim 22 or 23, **characterized in that** the guide (66) comprises at least one through-opening (68, 70, 72, 74).

25. Implant set in accordance with claim 24, **characterized in that** the at least one through-opening (68, 70, 72, 74), in the connected position in which the targeting device (12) is connected to an implant (14, 16, 18, 20), is oriented in alignment with the at least one bore (126, 128, 130, 132) and/or the at least one threaded bore of the implant (14, 16, 18, 20).

26. Implant set in accordance with claim 24 or 25, **characterized in that** a through-opening longitudinal axis (69, 71, 73, 75) defined by the at least one through-opening (68, 70, 72, 74) defines, in the connected position, a bore longitudinal axis (127, 129, 131, 133) of the at least one bore (126, 128, 130, 132) and/or of the at least one threaded bore.

27. Implant set in accordance with any one of claims 24 to 26, **characterized in that** the at least one through-opening (68, 70, 72, 74) is in the form of a target bore (68, 70, 72, 74).

28. Implant set in accordance with any one of claims 24 to 27, **characterized in that** the guide (66) comprises at least one sleeve (82, 88, 90) for a drilling and/or milling tool.

29. Implant set in accordance with claim 28, **characterized in that** the at least one sleeve (82, 88, 90) is guided and/or held in the at least one through-opening (68, 70, 72, 74).

30. Implant set in accordance with claim 28 or 29, **characterized in that** the at least one sleeve (82, 88, 90) is releasably connectable to the targeting device (12).

31. Implant set in accordance with any one of claims 28 to 30, **characterized in that** the at least one sleeve (82, 88, 90) is mounted for displacement on the targeting device (12).

32. Implant set in accordance with any one of claims 17 to 31, **characterized in that** only a single targeting device (12) is provided, which is connectable to all implants (14, 16, 18, 20) of the implant set (10) directly and without the use of intermediate pieces, attachments and/or adapters.

33. Implant set in accordance with any one of claims 17 to 32, **characterized in that** the second connecting device (36) is configured so as to correspond to the first connecting device (114), and **in that** in the connected position, the first and second connecting devices (114, 36) are connected to each other in a force-locking and/or form-locking manner.

34. Implant set in accordance with any one of claims 17 to 33, **characterized in that** the second connecting device (36) comprises at least one second connecting element (38) which, in the connected position, is in engagement with the first connecting element (116) in a force-locking and/or form-locking manner.

35. Implant set in accordance with claim 34, **characterized in that** the first or the second connecting element (38) comprises an externally threaded section (42), and **in that** the corresponding other connecting element (116) comprises an internally threaded section (118) corresponding to the externally threaded section (42).

36. Implant set in accordance with claim 35, **characterized in that** the first or the second connecting element (116) is in the form of a threaded bolt (116) having an elongated head portion (40) on which the externally threaded section (42) is arranged.

37. Implant set in accordance with any one of claims 34 to 36, **characterized in that** the targeting device (12) comprises a connecting element guide (46) for guiding and/or mounting the second connecting element (38).

38. Implant set in accordance with claim 37, **characterized in that** the connecting element guide (46) is formed coaxially or substantially coaxially with a longitudinal axis (24) defined by the first end area (32).

39. Implant set in accordance with claim 37 or 38, **characterized in that** the connecting element guide (46) comprises at least one stop (50) on which a part (40) of the second connecting element (38) is supported in the connected position.

40. Implant set in accordance with any one of claims 17 to 39, **characterized in that** the targeting device (12) and an implant (14, 16, 18, 20) connected thereto are held, in a connected position, in a force-locking and/or form-locking manner on each other.

41. Implant set in accordance with claim 40, **characterized in that** the targeting device (12) and one of the at least two implants (14, 16, 18, 20) are held, in the connected position, clamped to each other.

42. Implant set in accordance with any one of the preceding claims, **characterized in that** the implants (14, 16, 18, 20) of the implant set (10) are medullary nails.

## Revendications

1. Ensemble ou set d'implants (10) comprenant au moins deux implants (14, 16, 18, 20) de forme différente, qui présentent chacun un corps en tige (15, 17, 19, 21) comportant une extrémité proximale et distale, ainsi qu'un premier dispositif de liaison (114, 114a, 114b, 114c) prévu à l'extrémité proximale et destiné à la liaison avec un appareil de visée (12), le premier dispositif de liaison (114, 114a, 114b, 114c) définissant une direction de liaison, **caractérisé en ce qu'**au moins deux implants (14, 16, 18, 20) présentent des premiers dispositifs de liaison (114, 114a, 114b, 114c), qui, relativement à un axe longitudinal (104, 104a, 104b, 104c) défini par une zone d'extrémité proximale (102) du corps en tige (15, 17, 19, 21) respectif, définissent des directions de liaison d'inclinaison différente.

2. Set d'implants selon la revendication 1, **caractérisé en ce que** des directions de liaison d'implants différents (14, 16, 18, 20) sont inclinées par rapport aux axes longitudinaux (104, 104a, 104b, 104c) définis par les zones d'extrémité proximales (102) respectives, d'un angle (124, 124a, 124b, 124c) situé dans une plage de 0° à 40°.

3. Set d'implants selon la revendication 1 ou la revendication 2, **caractérisé en ce que** des directions de liaison d'implants différents (14, 16, 18, 20) se distinguent respectivement par rapport aux axes longitudinaux définis par les zones d'extrémité proximales (102) respectives, d'un angle (124, 124a, 124b, 124c) situé dans une plage de 3° à 8°.

4. Set d'implants selon l'une des revendications précédentes, **caractérisé en ce que** les corps en tige (15, 17, 19, 21) desdits au moins deux implants (14, 16, 18, 20) de forme différente, sont d'une longueur différente.

5. Set d'implants selon l'une des revendications précédentes, **caractérisé en ce que** les corps en tige (15, 17, 19, 21) desdits au moins deux implants (14, 16, 18, 20) de forme différente, sont de courbure différente.

6. Set d'implants selon l'une des revendications précédentes, **caractérisé en ce que** les corps en tige (15, 17, 19, 21) desdits au moins deux implants (14, 16, 18, 20) de forme différente, définissent des angles CCD différents (angle du col de fémur : centrum-collum-diaphyse).

7. Set d'implants selon l'une des revendications précédentes, **caractérisé en ce que** lesdits au moins deux implants (14, 16, 18, 20) présentent chacun au moins un logement de réception d'élément de fixation (126, 128, 130, 132).

8. Set d'implants selon la revendication 7, **caractérisé en ce que** sont prévus au moins deux logements de réception d'élément de fixation (126, 128, 130, 132), qui sont orientés de manière identique l'un par rapport à l'autre pour lesdits au moins deux implants (14, 16, 18, 20) de forme différente.

9. Set d'implants la revendication 7 ou la revendication 8, **caractérisé en ce que** ledit au moins un logement de réception d'élément de fixation (126, 128, 130, 132) est prévu à une zone d'extrémité proximale et/ou distale (102) du corps en tige (15, 17, 19, 21).

10. Set d'implants selon l'une des revendications précédentes, **caractérisé en ce que** le premier dispositif de liaison (114, 114a, 114b, 114c) comprend au moins un premier élément de liaison (116).

11. Set d'implants selon la revendication 10,
**caractérisé en ce que** ledit au moins un élément de liaison (116) comprend un trou borgne (120) muni d'un taraudage ou filetage intérieur (118).

12. Set d'implants selon la revendication 10 ou la revendication 11, **caractérisé en ce qu'**un axe longitudinal (24) défini par le premier élément de liaison (116) est incliné, par rapport à un axe longitudinal (104, 104a, 104b, 104c) défini par la zone d'extrémité proximale (102) du corps en tige (15, 17, 19, 21) respectif, de manière différente pour au moins deux implants (14, 16, 18, 20).

13. Set d'implants selon la revendication 12,
**caractérisé en ce que** les axes longitudinaux (24) définis par les premiers éléments de liaison (116) des implants (14, 16, 18, 20) sont inclinés par rapport aux axes longitudinaux (104, 104a, 104b, 104c) définis par les zones d'extrémité proximales (102) respectives, d'un angle (124, 124a, 124, 124c) situé dans une plage de 0° à 40°.

14. Set d'implants selon la revendication 12 ou la revendication 13, **caractérisé en ce que** les axes longitudinaux (24) définis par les premiers éléments de liaison (116) d'implants différents (14, 16, 18, 20) se distinguent respectivement par rapport aux axes longitudinaux (104, 104a, 104b, 104c) définis par les zones d'extrémité proximales (102) respectives, d'un angle (124, 124a, 124, 124c) situé dans une plage de 3° à 8°.

15. Set d'implants selon l'une des revendications précédentes, **caractérisé en ce qu'**il est réalisé un contour (108) du corps en tige (15, 17, 19, 21), qui est dirigé vers l'extrémité proximale, ce contour (108) présentant deux zones de surface (110, 112) inclinées l'une par rapport à l'autre d'un angle d'inclinaison (60).

16. Set d'implants selon la revendication 15,
**caractérisé en ce que** l'angle d'inclinaison (60) présente une valeur située dans une plage de 90° à 150°.

17. Set d'implants selon l'une des revendications précédentes, **caractérisé par** un appareil de visée (12) avec un deuxième dispositif de liaison (36) qui peut être relié au premier dispositif de liaison (114), dans une position de liaison avec lesdits au moins deux implants (14, 16, 18, 20) du set d'implants (10).

18. Set d'implants selon la revendication 17,
**caractérisé en ce qu'**un contour (108) du corps en tige (15, 17, 19, 21), qui est dirigé vers l'extrémité proximale, est réalisé de manière à correspondre à une zone d'appui (54) prévue sur l'appareil de visée (12).

19. Set d'implants selon la revendication 17 ou la revendication 18, **caractérisé en ce que** l'appareil de visée (12) présente une première zone d'extrémité (32) et une deuxième zone d'extrémité (34), et **en ce que** le deuxième dispositif de liaison (36) est prévu au niveau de la première zone d'extrémité (32).

20. Set d'implants selon la revendication 19,
**caractérisé en ce que** l'appareil de visée (12) est d'une configuration sensiblement en forme de U comprenant une première branche (22) englobant la première zone d'extrémité (32), une deuxième branche (26) englobant la deuxième zone d'extrémité (34), et un tronçon de liaison (30) reliant les branches (22, 26).

21. Set d'implants selon la revendication 20, **caractérisé en ce que** la première branche et la deuxième branche (22, 26) sont orientées de manière parallèle ou sensiblement parallèle l'une à l'autre.

22. Set d'implants selon l'une des revendications 17 à 21, **caractérisé en ce que** l'appareil de visée (12) comprend au moins un guidage (66) destiné à guider un outil de perçage et/ou de fraisage.

23. Set d'implants selon la revendication 22, **caractérisé en ce que** le guidage (66) et prévu au niveau de la deuxième zone d'extrémité (34).

24. Set d'implants selon la revendication 22 ou la revendication 23, **caractérisé en ce que** le guidage (66) comprend au moins un passage (68, 70, 72, 74).

25. Set d'implants selon la revendication 24, **caractérisé en ce que** ledit au moins un passage (68, 70, 72, 74), dans la position de liaison dans laquelle l'appareil de visée (12) est relié à un implant (14, 16, 18, 20), est orienté de manière alignée avec ledit au moins un alésage (126, 128, 130, 132) et/ou ledit au moins un alésage taraudé de l'implant (14, 16, 18, 20).

26. Set d'implants selon la revendication 24 ou la revendication 25, **caractérisé en ce qu'**un axe longitudinal de passage (69, 71, 73, 75) défini par ledit au moins un passage (68, 70, 72, 74), définit, dans la position de liaison, un axe longitudinal de perçage (127, 129, 131, 133) dudit au moins un alésage ou perçage (126, 128, 130, 132) et/ou dudit au moins un alésage taraudé.

27. Set d'implants selon l'une des revendications 24 à 26, **caractérisé en ce que** ledit au moins un passage (68, 70, 72, 74) est réalisé en tant qu'alésage ou perçage de visée (68, 70, 72, 74).

28. Set d'implants selon l'une des revendications 24 à 27, **caractérisé en ce que** le guidage (66) comprend au moins une douille ou un canon de perçage (82, 88, 90) pour un outil de perçage et/ou de fraisage.

29. Set d'implants selon la revendication 28, **caractérisé en ce que** ladite au moins une douille ou ledit au moins un canon de perçage (82, 88, 90) est guidé et/ou maintenu dans ledit au moins un passage (68, 70, 72, 74).

30. Set d'implants selon la revendication 28 ou la revendication 29, **caractérisé en ce que** ladite au moins une douille ou ledit au moins un canon de perçage (82, 88, 90) peut être relié de manière amovible à l'appareil de visée (12).

31. Set d'implants selon l'une des revendications 28 à 30, **caractérisé en ce que** ladite au moins une douille ou ledit au moins un canon de perçage (82, 88, 90) est monté coulissant sur l'appareil de visée (12).

32. Set d'implants selon l'une des revendications 17 à 31, **caractérisé en ce qu'**il n'est prévu qu'un seul appareil de visée (12) qui peut être relié à tous les implants (14, 16, 18, 20) du set d'implants (10), de manière directe et sans utilisation de pièces intermédiaires, de pièces rapportées et/ou d'adaptateurs.

33. Set d'implants selon l'une des revendications 17 à 32, **caractérisé en ce que** le deuxième dispositif de liaison (36) est réalisé de manière à correspondre au premier dispositif de liaison (114), et **en ce que** dans la position de liaison, le premier et le deuxième dispositif de liaison (114, 36) sont reliés mutuellement par adhérence et/ou complémentarité de formes.

34. Set d'implants selon l'une des revendications 17 à 33, **caractérisé en ce que** le deuxième dispositif de liaison (36) comprend au moins un deuxième élément de liaison (38) qui, dans la position de liaison, est en prise par adhérence et/ou complémentarité de formes avec le premier élément de liaison (116).

35. Set d'implants selon la revendication 34, **caractérisé en ce que** le premier ou le deuxième élément de liaison (38) comprend un tronçon de filetage extérieur (42), et **en ce que** l'autre élément de liaison correspondant (116) présente un tronçon de filetage intérieur (118) correspondant au tronçon de filetage extérieur (42).

36. Set d'implants selon la revendication 35,
**caractérisé en ce que** le premier ou le deuxième élément de liaison (116) est réalisé sous la forme d'une broche à visser (116), qui présente une partie de tête (40) allongée sur laquelle est agencé le tronçon de filetage extérieur (42).

37. Set d'implants selon l'une des revendications 34 à 36, **caractérisé en ce que** l'appareil de visée (12) comprend un guidage d'élément de liaison (46) pour guider et/ou assurer le montage du deuxième élément de liaison (38).

38. Set d'implants selon la revendication 37,
**caractérisé en ce que** le guidage d'élément de liaison (46) est réalisé de manière coaxiale ou sensiblement coaxiale à un axe longitudinal (24) défini par la première zone d'extrémité (32).

39. Set d'implants selon la revendication 37 ou la revendication 38, **caractérisé en ce que** le guidage d'élément de liaison (46) présente au moins une butée (50) contre laquelle s'appuie une partie (40) du deuxième élément de liaison (38), dans la position de liaison.

40. Set d'implants selon l'une des revendications 17 à 39, **caractérisé en ce que** l'appareil de visée (12) et un implant (14, 16, 18, 20) qui y est relié, sont, dans une position de liaison, maintenus l'un contre l'autre par adhérence et/ou complémentarité de formes.

41. Set d'implants selon la revendication 40,
**caractérisé en ce que** l'appareil de visée (12) et l'un desdits au moins deux implants (14, 16, 18, 20) sont, dans la position de liaison, maintenus l'un contre l'autre par serrage.

42. Set d'implants selon l'une des revendications précédentes, **caractérisé en ce que** les implants (14, 16, 18, 20) du set d'implants (10) sont des clous médullaires.
